# EUROPEAN PATENT APPLICATION

(11) **EP 4 708 192 A1**
(43) Date of publication of application: **11.03.2026**
(21) Application number: 24800274.3
(22) Date of filing: 03.05.2024
(51) Int. Cl.: G06T 7/00, G06T 7/62, G06F 21/32, G16H 30/00, G16H 10/60, G16H 50/20, G16H 40/20, G16H 80/00

(54) **APPARATUS AND METHOD FOR ANALYZING MEDICAL EXAMINATION RESULT IMAGES**

(30) Priority: 04.05.2023 KR 20230058706; 02.05.2024 KR 20240058514
(71) Applicant: Seoul National University Hospital, Seoul 03080 (KR)
(72) Inventor: KIM, Joonghee, Seongnam-si, Gyeonggi-do 13620 (KR)
(74) Representative: Dentons Patent Solutions Rechtsanwaltsgesellschaft mbH
(86) International application number: PCT/KR2024/006043
(87) International publication number: WO 2024/228593

(57) **Abstract**

Disclosed are a medical examination result image analysis apparatus and a medical examination result image analysis method performed thereby. The method comprises the steps of: selecting, by a processor, by using an artificial neural network-based detection model, a test area to be provided for analysis from among medical test result images displayed on a screen; and analyzing, by the processor, the selected test area by using an artificial neural network-based analysis model, providing results of the analysis, wherein the medical test result images may include one or more of an electrocardiogram image and a chest X-ray image.

## Description

### Technical Field

The disclosure relates to an apparatus and a method for analyzing medical test (examination) result images.

### Cross-Reference to Related Applications

This application claims priority to Korean Provisional Patent Application No. 10-2023-0058706, filed on May 4, 2023, and Korean Patent Application No. 10-2024-0058514, filed on May 2, 2024, the disclosures of which are incorporated herein by reference.

### Background Art

An electrocardiogram (ECG) is a recording of the electrical activity generated in the myocardium according to the heartbeat. The ECG is a very simple test that provides immediate results and is also useful for continuous patient monitoring. Additionally, chest X-ray images showing information about thoracic structures are widely utilized because they can be easily obtained.

Analysis of medical images, such as ECG images or chest X-ray images, plays an important role in the medical field for purposes of diagnosing diseases, particularly heart-related diseases, establishing treatment plans, and monitoring diseases.

The use of advanced computer vision technologies, particularly deep learning-based image analysis models, may contribute to improving the accuracy and efficiency of medical image analysis. However, specialized methods are still required to effectively select and analyze various types of medical images, such as ECG images or chest X-ray images, and to provide results with high user convenience.

### Detailed Description of the Invention

### Technical Solution

In embodiments of the present application, there are provided an apparatus and a method for analyzing test result images, wherein test areas are automatically selected and analyzed from medical test result images, such as electrocardiogram (ECG) images and/or chest X-ray images, and then providing a natural language description and/or visualized digital biomarker test result.

### Summary of the Invention

A method for analyzing medical test result images according to an embodiment is performed by an apparatus for analyzing medical test result images, the apparatus comprising one or more processors and one or more memories for storing instructions executed by the one or more processors, the method comprising: selecting, by the processor, by using an artificial neural network-based detection model, a test area to be provided for analysis from among medical test result images displayed on a screen; and analyzing, by the processor, the selected test area by using an artificial neural network-based analysis model, and providing results of the analysis, wherein the medical test result images include one or more of an electrocardiogram (ECG) image and a chest X-ray image.

The detection model may be configured to generate one or more test area candidate boundary boxes and output a tuple including one or more of target test class information and candidate boundary box information.

The tuple may further include a confidence score (S), which is a quantitative value indicating whether the class information and the candidate boundary box information correspond to target class or target boundary box information.

The screen may be obtained by capturing the medical test result images, and the method may include calculating a distance (D) between the center of the screen and each of centers of a plurality of candidate boundary boxes, and selecting one of the plurality of candidate boundary boxes as the test area of the medical test result images based on the confidence score (S) and the distance (D).

The method may include selecting one of the plurality of candidate boundary boxes as a test area of the medical test result images based on a result value (S') of a function [f(S, D)] having the confidence score (S) and the distance (D) as variables, wherein the function [f(S, D)] may be selected as any one of S, 1/D, and S/D.

The distance (D) may be calculated by one or more of the Euclidean distance, the Manhattan distance, and the Chebyshev distance.

The class information may include an index value of a target test, and the candidate boundary box information may include a numerical value or a combination of numerical values capable of defining the boundary of the box.

The method may include changing areas of the plurality of candidate boundary boxes based on the user's drag and resize input.

The method may include providing results of the analysis corresponding to the target test using an analysis model assigned to each target test class.

The method may further include providing one or more of a test date, a test time, patient identification information, and a patient information memo in addition to the test area.

The method may include further providing one or more of a test name, a pending display, and a completion notification when providing the results of the analysis.

The method may include generating a natural language description based on a biomarker value corresponding to the results of the analysis while providing the natural language description as the results of the analysis.

The natural language description may be a rule-based description or a large language model-based description.

The rule-based description may be automatically output when the predetermined biomarker value corresponding to the results of the analysis is within a set range.

The large language model-based description may be output after generating a prompt based on the biomarker value corresponding to the results of the analysis and inputting the prompt to a large language model.

The large language model-based description may be generated by generating, according to predefined rules, at least one of the clinical significance of the biomarker and the risk pair derived from measurement values.

The prompt may further include additional prompts for describing an answer to the associated request content.

The providing of the results of the analysis may include providing digital biomarker test results in a visualized form.

The digital biomarker test results may include one or more of names of the digital biomarkers, values of the digital biomarkers, and a distribution of the values of the digital biomarkers.

The digital biomarker test results may include one or more of a type of cardiac rhythm and a probability of the cardiac rhythm corresponding to the type.

The digital biomarker test results may further include a left ventricular ejection fraction.

The digital biomarker test results may further provide an ECG thumbnail image, and the method may further include providing an original ECG image in response to a request for outputting the thumbnail image.

The method may further include requesting consultation among medical professionals with respect to the medical test results, wherein the requesting of the consultation may include: providing a medical professional's buddy list; displaying a consultation availability status based on a stored work schedule of the medical professional's buddy in the list; and transmitting a consultation request to the requested medical professional's buddy in response to a consultation request input and receiving a response regarding the consultation, wherein the work schedule is electronically updatable. When an update of the work schedule is automatically detected by the processor, the consultation availability status is updated.

The method may further include providing an activation indicator indicating whether consultation with the medical professional's buddy is available.

The method may further include providing a user interface for modifying the work schedule.

The consultation request input may include one or more of patient information and consultation request items.

The providing of the analysis may include matching an input first test result with a preset first test biomarker pool; additionally confirming another second test biomarker when the matching result corresponds to predefined conditions; analyzing the first test biomarker and the second test biomarker and combining results; and outputting the combined results.

The first test is an ECG, and the second test is a chest X-ray. The preset first test or second test biomarker pool may include biomarkers related to one or more of left ventricular dysfunction, right ventricular dysfunction, pulmonary arterial hypertension, pulmonary edema, pericardial effusion, aortic dissection, aortic dilatation, (left/right) atrial hypertrophy, (left/right) ventricular hypertrophy, coronary artery calcification/elevated calcium score, coronary artery disease, hypertrophic/dilated/stress cardiomyopathy, and valve stenosis/insufficiency/replacement (AV, MV, TV, PV).

When the ECG biomarker is STEMI/ACS, the corresponding chest X-ray biomarker is Aortic dissection. When the ECG biomarker is LV dysfunction or pulmonary edema, the corresponding chest X-ray biomarker is LV dysfunction or pulmonary edema. When the ECG biomarker is RV dysfunction or pulmonary hypertension, the corresponding chest X-ray biomarker is RV dysfunction or pulmonary hypertension or pulmonary embolism. When the ECG biomarker is large pericardial effusion, the corresponding chest x-ray biomarker may be large pericardial effusion.

The apparatus for analyzing medical test results according to an embodiment may include one or more processors, and one or more memories for storing instructions executed by the one or more processors, wherein the apparatus analyzes medical test result images by the aforementioned method. The one or more processors are configured to provide a first UI screen for obtaining medical test result images, and provide a second UI screen for displaying one or more of a target class and a boundary box for selecting a test area from the obtained medical test result images.

### Effects of the Invention

In one aspect, according to embodiments of the present application, a test area may be accurately and effectively obtained from medical test result images, such as ECG images and/or chest X-ray images, to be provided for analysis.

In another aspect, according to embodiments of the present application, medical image results of the analysis may be provided with high user convenience by automatically selecting and analyzing the acquired test area and then providing natural language descriptions and/or digital biomarker test results in a schematic form.

The effects of the present application are not limited to the effects mentioned above, and other effects not mentioned will be clearly understood by those skilled in the art from the disclosure of the claims.

### Brief Description of the Drawings

To more clearly illustrate embodiments of the present application, the drawings necessary for describing the embodiments are briefly introduced below. It should be understood that the following drawings are for the purpose of illustrating the embodiments of the present specification and not for the purpose of limiting the same. Furthermore, for clarity of description, some elements may be shown in the following drawings with various modifications, such as exaggeration, omission, and the like, applied thereto.
FIG. 1 is a screen illustrating the configuration of an apparatus for analyzing test results, in an embodiment of the present application.
FIG. 2A is a screen of an initial input user interface of the apparatus for analyzing test results, in an embodiment of the present application.
FIG. 2B is a screen illustrating selection of an electrocardiogram (ECG) image test area of the apparatus for analyzing test results, in an embodiment of the present application.
FIG. 2C is another example illustrating selection of the ECG image test area of the apparatus for analyzing test results, in an embodiment of the present application.
FIG. 2D is a screen illustrating area modification, by drag input, of a boundary box of the apparatus for analyzing test results, in an embodiment of the present application.
FIG. 2E is a screen illustrating selection of a chest X-ray image test area of the apparatus for analyzing test results, in an embodiment of the present application.
FIG. 2F is a standby screen of the user interface of the apparatus for analyzing test results, after analysis initiation, in an embodiment of the present application.
FIG. 2G is an analysis completion screen of the user interface of the apparatus for analyzing test results, in an embodiment of the present application.
FIG. 2H is an output screen of the natural language description of the apparatus for analyzing test results, in an embodiment of the present application.
FIG. 2I is another output screen of the natural language description of the apparatus for analyzing test results, in an embodiment of the present application.
FIG. 2J is a screen of a digital biomarker test result image of the apparatus for analyzing test results, in an embodiment of the present application.
FIG. 2K is another example of the digital biomarker test result image of the apparatus for analyzing test results, in an embodiment of the present application
FIG. 2L is a screen of other output information images of the apparatus for analyzing test results, in an embodiment of the present application.
FIGS. 3A to 3E are screens illustrating a consultation function in the user interface of the apparatus for analyzing test results, in an embodiment of the present application.
FIGS. 4A and 4B are screens where the apparatus for analyzing test results additionally instructs evaluation of chest radiographic biomarkers after evaluation of ECG biomarkers, in an embodiment of the present application.

### Detailed Description of The Invention

Hereinafter, some embodiments of the present application will be described in detail with reference to example drawings. In assigning reference numerals to components in each drawing, the same components may have the same reference numerals as much as possible even if they are shown in different drawings. Furthermore, in describing the present embodiments, when it is determined that a detailed description of related known configurations or functions may obscure the gist of the technical concept, the detailed description may be omitted.

### Definition of Terms

When terms such as "comprise," "have," and "consist of," are used in this specification, other portions may be added unless "only" is used. When a component is expressed in a singular form, it may include a plural form unless explicitly stated otherwise.

Furthermore, in describing components of the present application, terms such as first, second, A, B, (a), (b), and the like may be used. Unless otherwise specified, these terms are merely for distinguishing the component from other components. The essence, sequence, order, or number of the corresponding components is not limited by such terms.

In this specification, terms such as "unit", "module", "apparatus", or "system" are intended to refer to a combination of hardware and software driven by such hardware. For example, the hardware may be a data processing device including a central processing unit (CPU), graphics processing unit (GPU), or other processor. Additionally, the software may refer to a running process, an object, an executable, a thread of execution, a program, or the like.

In this specification, a boundary box or a candidate boundary box refers to a region for selecting a test area to be analyzed on a screen containing test result images. It is referred to as a boundary box because the boundary of the box is defined by the boundary of the corresponding region.

In this specification, "test area" refers to a selected analysis target region on a screen containing test result images.

In this specification, "class" refers to a display index of a target test, and the index may be represented, for example, by a number.

In this specification, "medical test image" includes, but is not limited to, an electrocardiogram (ECG) image and/or a chest X-ray image.

In this specification, acquisition of medical test images may include acquisition of images by an imaging apparatus of a user terminal or from a storage device of the user terminal.

In this specification, the biomarker refers to a digital biomarker.

### Description of Exemplary Embodiments

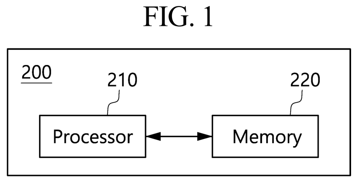

FIG. 1 is a screen illustrating the configuration of an apparatus for analyzing test results, in an embodiment of the present application.

Referring to FIG. 1, the apparatus 200 according to an embodiment includes one or more processors 210 and one or more memories 220 that store instructions to be executed by the one or more processors 210.

The apparatus acquires medical test images as medical test results, selects and analyzes a test area from the acquired medical test images, and provides results of the analysis.

Specifically, the apparatus may include a first UI screen including, for example, an imaging button for acquiring a medical test image and a second UI screen for displaying one or more of a target class and a boundary box for selecting the test area from the acquired medical test image.

The processor of the apparatus may provide results of the analysis from medical test result images as follows.

In an embodiment, the processor may perform the steps of selecting the test area to be provided for analysis from the medical test result image displayed on the screen using an artificial neural network-based detection model, and analyzing the selected test area with the artificial neural network-based analysis model to provide results of the analysis.

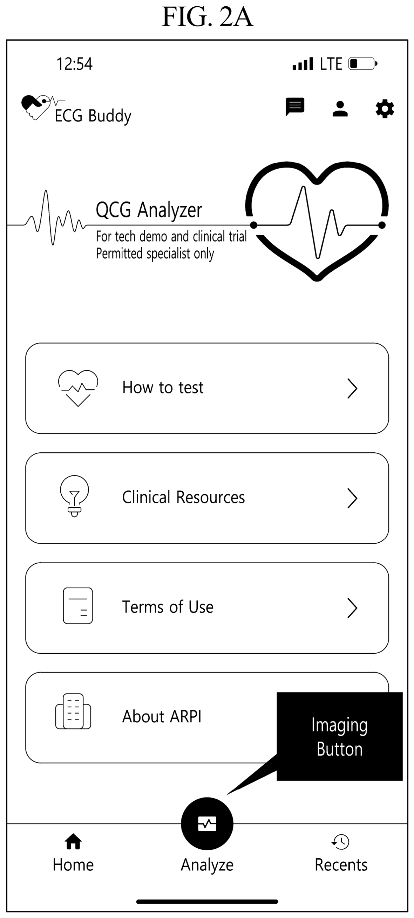

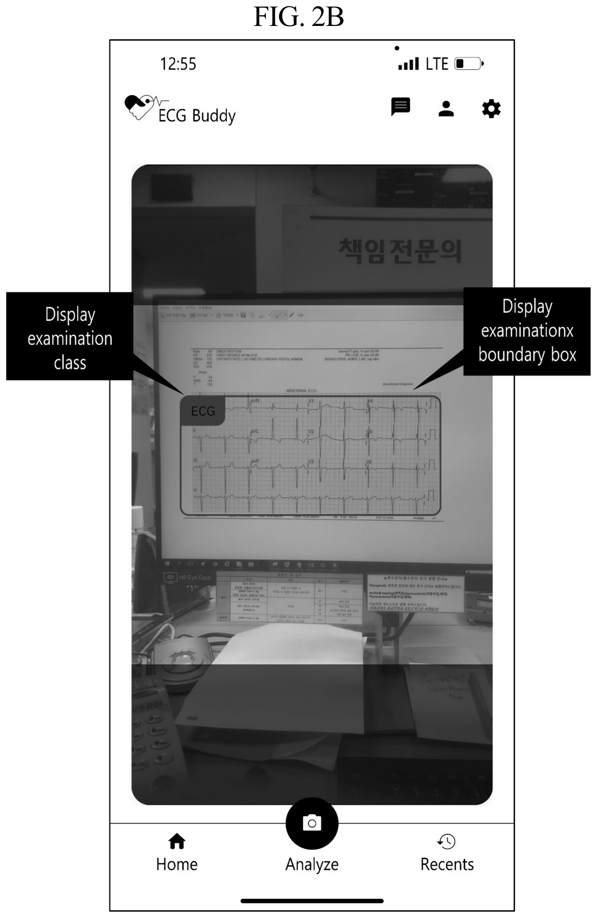

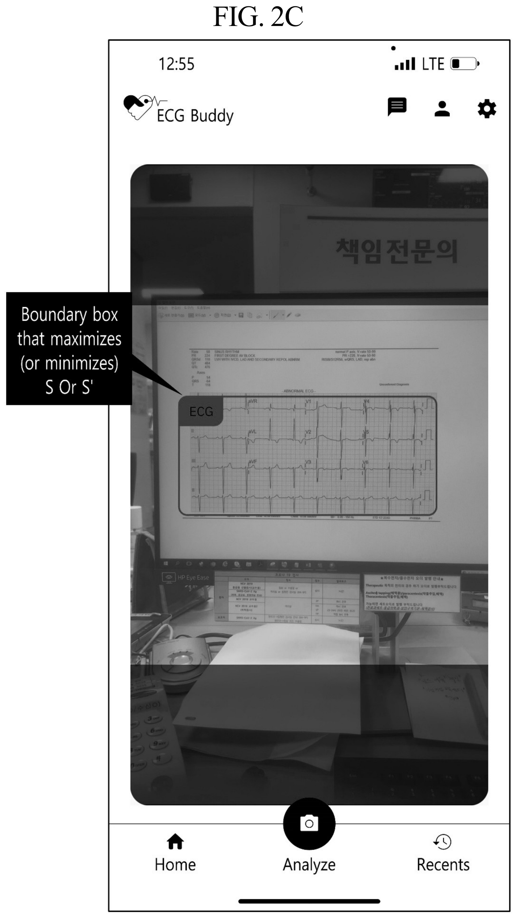

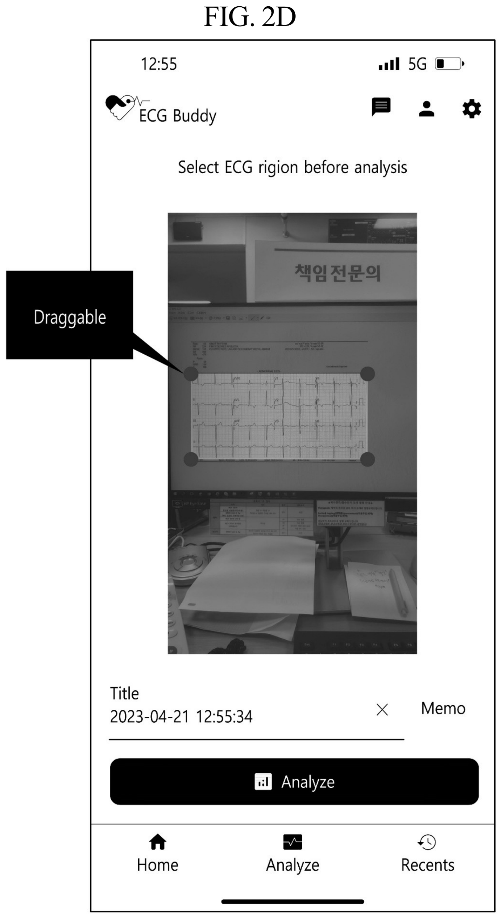

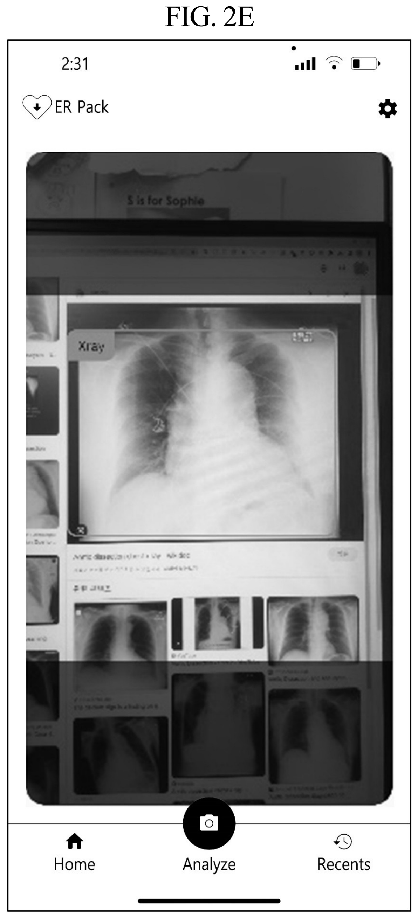

FIG. 2A is a screen of an initial input user interface of the apparatus for analyzing test results. FIG. 2B is a screen illustrating selection of an ECG image test area of the apparatus for analyzing test results. FIG. 2C is another example illustrating selection of the ECG image test area of the apparatus for analyzing test results. FIG. 2D is a screen illustrating area modification, by drag input, of a boundary box of the apparatus for analyzing test results. FIG. 2E is a screen illustrating selection of a chest X-ray image test area of the apparatus for analyzing test results.

As shown in FIG. 2A, when a user presses an imaging button or an analysis button on an initial input user interface screen displayed on a user terminal display, the user may newly capture medical test result images, such as ECG images and/or chest X-ray images, or call and display recent medical test result images stored in a storage device of the user terminal.

As shown in FIGS. 2B and 2C, the test area to be provided for analysis is selected from the captured or called medical test result images using the artificial neural network-based detection model. The detection model generates one or more test area candidate boundary boxes and outputs a tuple containing one or more of target test class information and the candidate boundary box information.

The class information includes an index value of a target test, such as ECG and/or chest X-ray.

The candidate boundary box information includes a numerical value or a combination of numerical values that may define the boundary of the box. For example, the candidate boundary box information may include coordinate values of four vertices of a rectangle of a boundary box containing a target test area, such as a target test ECG image area and/or a target test chest X-ray image, or a combination of numerical values in another format that may define the same.

In an embodiment, to select a test area from the plurality of candidate boundary boxes, the tuple may further include a confidence score (S), which is a quantitative value regarding whether the class information and the candidate boundary box information correspond to target class or target boundary box information.

Additionally, a distance (D) between a center of a captured screen of the user terminal and a center of each of the plurality of candidate boundary boxes may be calculated, and based on the confidence score (S) and the distance (D), any one of the plurality of candidate boundary boxes may be selected as the test area of the medical test result images.

More specifically, the method selects one of the plurality of candidate boundary boxes as the test area of the medical test result images based on a result value (S') of a function [f(S, D)] having the confidence score (S) and the distance (D) as variables, wherein the function [f(S, D)] may be one selected from S, 1/D, and S/D, and corresponds to any type of function where S is maximized and D is minimized when the output of the f function becomes maximum or minimum.

In an embodiment, the distance (D) may be calculated by one or more of the Euclidean distance, the Manhattan distance, and the Chebyshev distance.

In an embodiment, a boundary box that minimizes or maximizes the aforementioned confidence score (S) and/or the result value (S') of the function [f(S, D)] may be selected as the test area from among the candidate boundary boxes.

As shown in FIG. 2D, in an embodiment of the present application, the areas of the plurality of candidate boundary boxes may be changed based on the user's drag and resize input. For the user's drag, drag points may be indicated, for example, at the four corners of the boundary box.

FIG. 2E illustrates selection of the test area from the chest X-ray image rather than the ECG image. Like the ECG image, the same detection algorithm may be used in the chest X-ray image to select the class and select the test area from the boundary box.

In the above-selected test area, one or more of a test date, a test time, patient identification information, such as a barcode or QR code, and a patient information memo (additional information about the patient other than patient identification information) may be further included and stored in the apparatus and provided for subsequent analysis.

Next, the analysis step is described.

In an embodiment, analysis may be initiated by cropping the area selected as the test area.

In an embodiment, the analysis model assigned to each target test class, such as ECG and/or chest X-ray, may be used to provide results of the analysis corresponding to the target test.

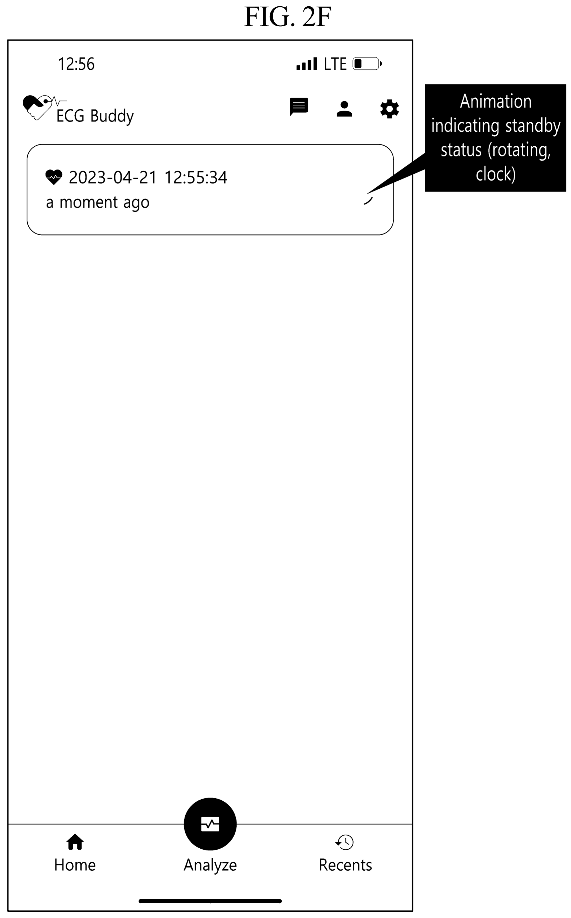

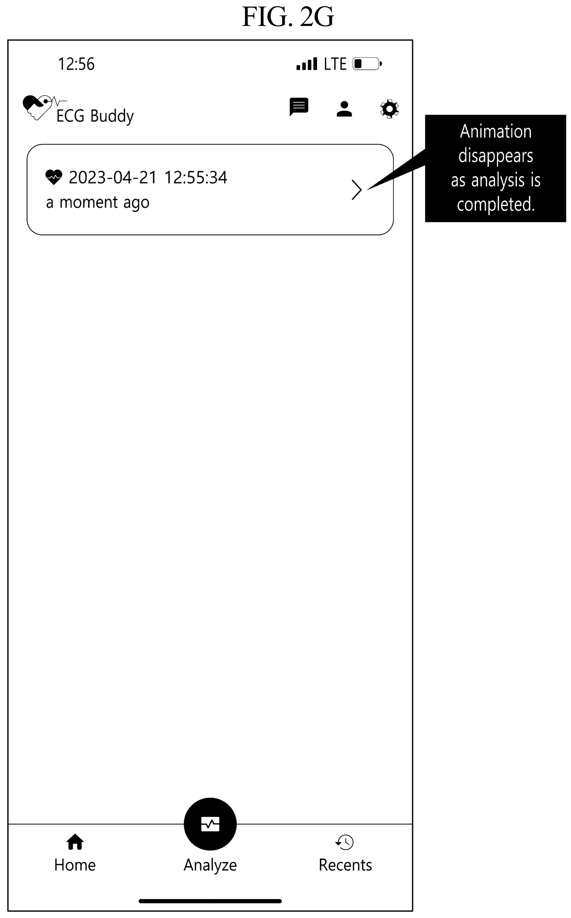

FIG. 2F is a standby screen of the user interface of the apparatus for analyzing test results, after analysis initiation, and FIG. 2G is an analysis completion screen of the user interface of the apparatus for analyzing test results.

As shown in FIGS. 2F and 2G, when providing the results of the analysis, one or more of a test name, a standby indicator, and a completion notification may be further provided.

For example, a standby display may be an animation display indicating that the system is in standby mode. When the test is completed, the animation may stop, an alarm sound may be output, and an alarm notification window may be generated.

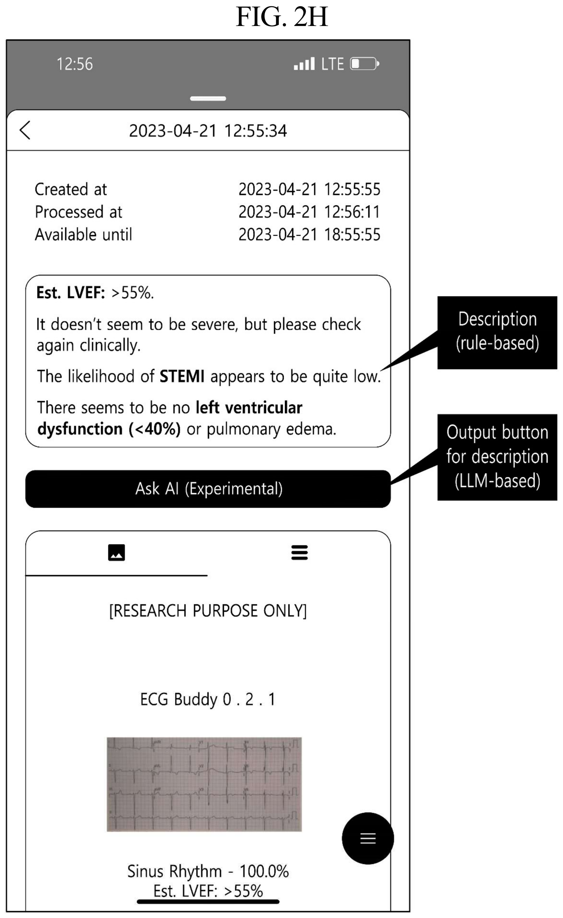

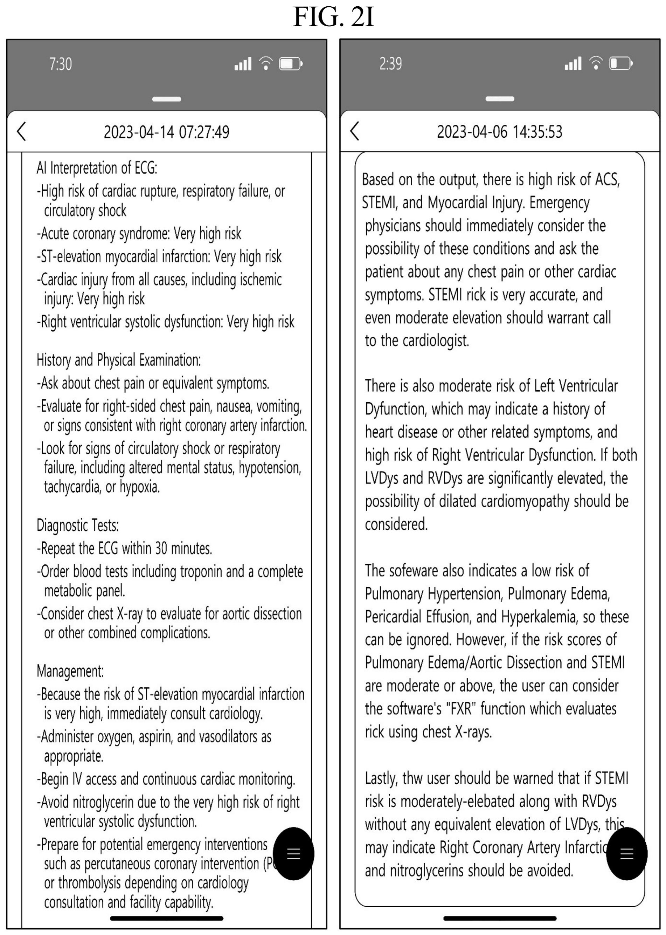

Meanwhile, FIG. 2H is an output screen of the natural language description of the apparatus for analyzing test results, and FIG. 2I is another output screen of the natural language description of the apparatus for analyzing test results.

In an embodiment, when providing results of the analysis, the natural language description may be provided as results of the analysis. The natural language description may be generated based on a biomarker value corresponding to the results of the analysis.

As an example, the natural language description may be a rule-based description or a large language model-based description.

The rule-based description is automatically output when the predetermined biomarker value corresponding to the results of the analysis falls within a set range, and the condition of the range may be a single condition or a combination of conditions.

The large language model-based description may be output by generating a prompt based on the biomarker value corresponding to the results of the analysis and inputting the prompt to the large language model.

In an embodiment, the generating of the prompt based on the biomarker value may include generating, according to predefined rules, at least one of the clinical significance of the biomarker and the risk pair derived from measurement values.

In addition, in an embodiment, the prompt may further include additional prompts for describing responses to associated request content.

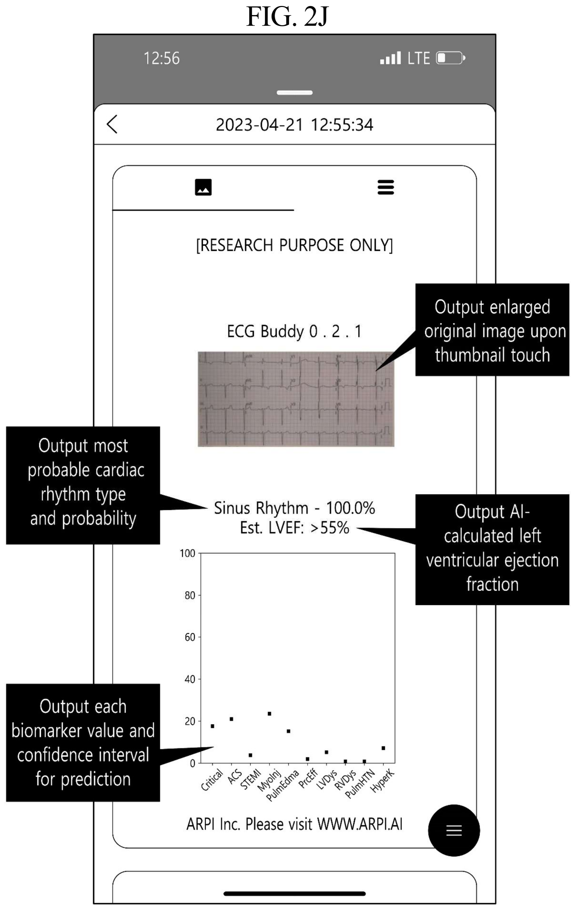

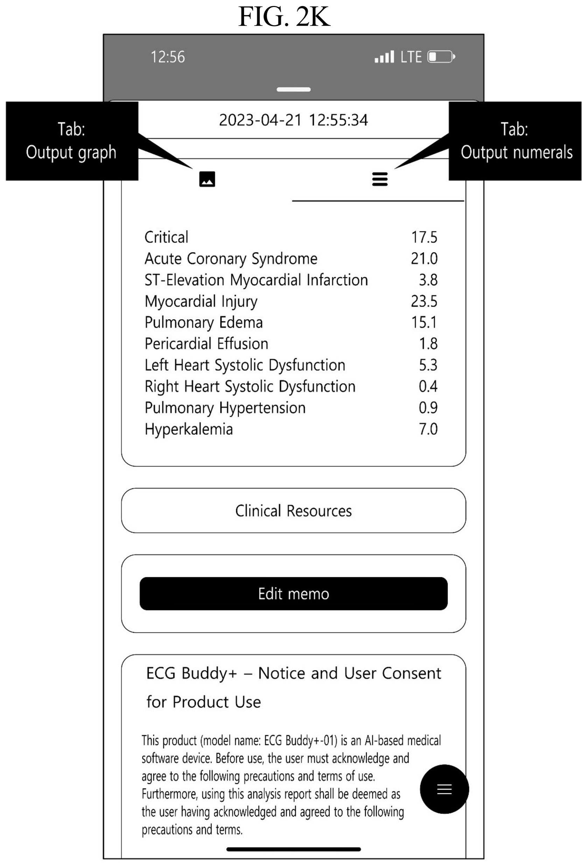

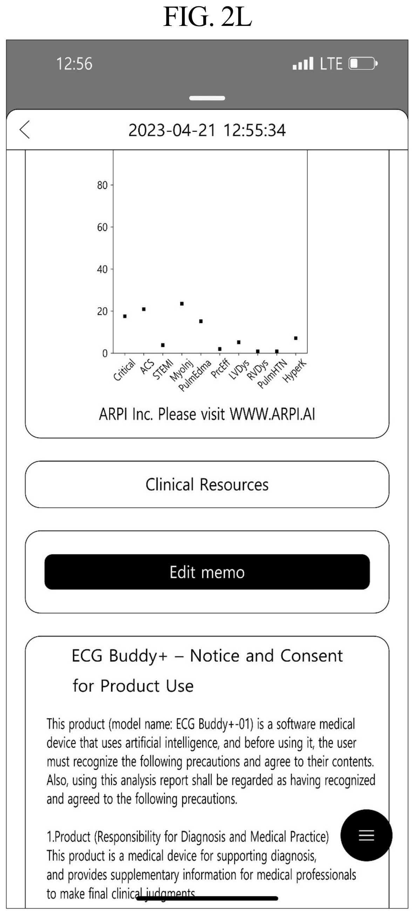

FIG. 2J is a screen of a digital biomarker test result image of the apparatus for analyzing test results. FIG. 2K is another example of the digital biomarker test result image of the apparatus for analyzing test results. FIG. 2L is a screen of other output information images of the apparatus for analyzing test results.

As shown in FIGS. 2J to 2L, when providing the results of the analysis, the digital biomarker test results may be provided in a visualized form.

The digital biomarker test results may include one or more of a name of the digital biomarker, a digital biomarker value, and distribution information of the corresponding value, such as a confidence interval.

As an example, the digital biomarker test results may include one or more of a type of cardiac rhythm and a probability that the cardiac rhythm corresponds to the type. For example, the digital biomarker test results may include a left ventricular ejection fraction.

In addition, in one example, the digital biomarker test results may further provide ECG thumbnail images and may further provide ECG original images in response to an output request for the thumbnail images.

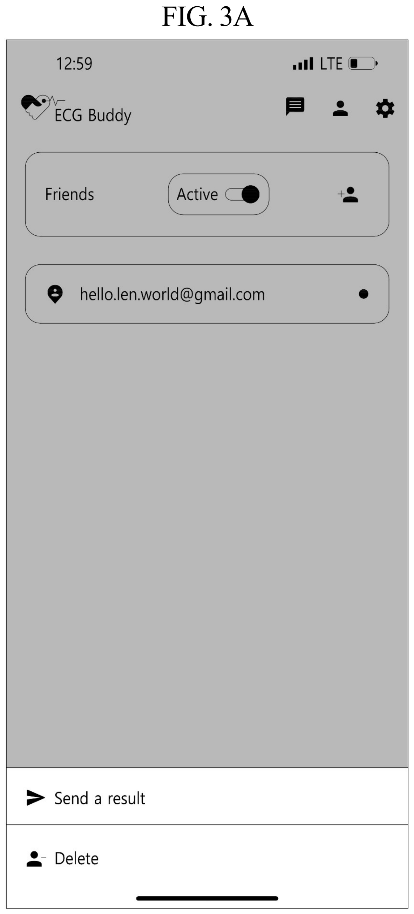

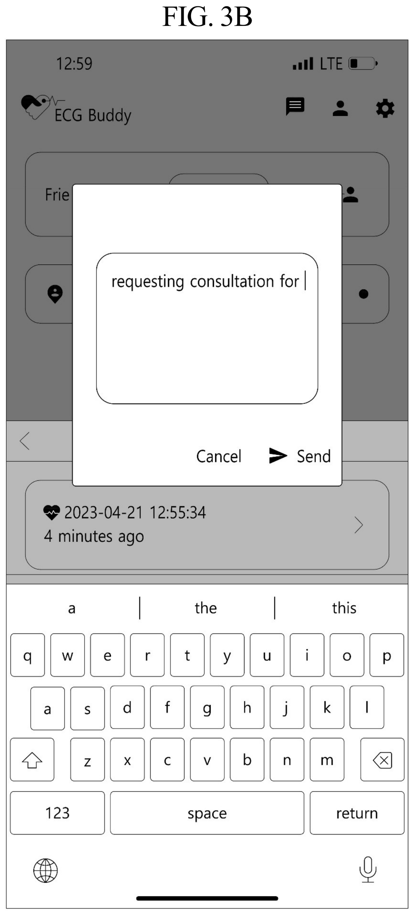

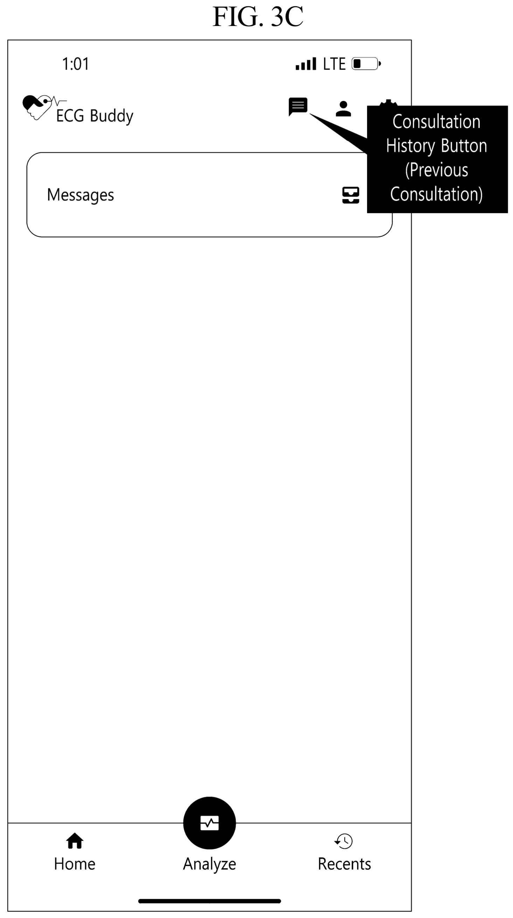

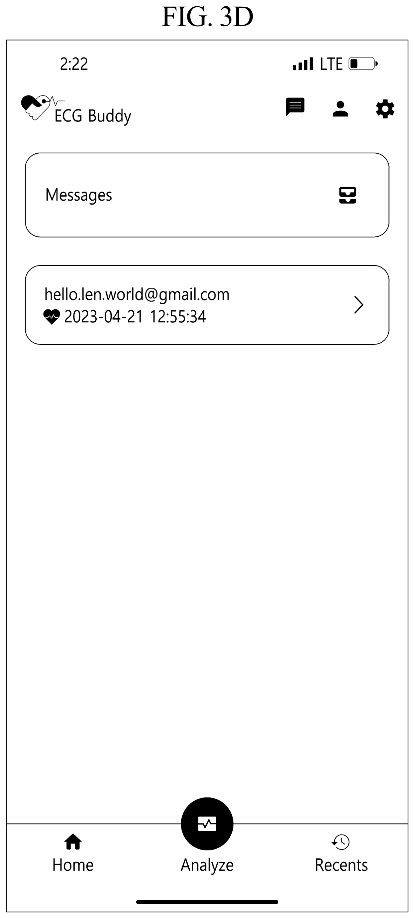

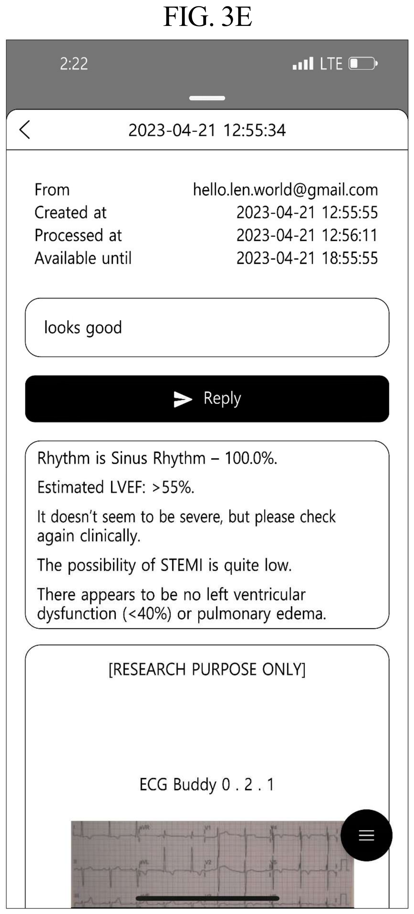

FIGS. 3A to 3E are screens illustrating a consultation function in the user interface of the apparatus for analyzing test results.

As shown in FIGS. 3A to 3E, the method for analyzing medical test result images of an embodiment of the present application may further include requesting consultation between medical professionals regarding medical test results.

The requesting of the consultation may include providing a medical professional's buddy list, indicating a consultation availability status based on a stored work schedule of the medical professional's buddy in the list, transmitting a consultation request to the requested medical professional's buddy in response to a consultation request input, and receiving a response regarding the consultation availability, and may further provide an activation indicator showing the consultation availability status of the medical professional's buddy.

The work schedule is electronically updatable, and the processor may automatically detect updates to the work schedule to refresh the consultation availability status. Additionally, a user interface for modifying the work schedule may be further provided.

The consultation request input may include one or more of consultation request items and patient information.

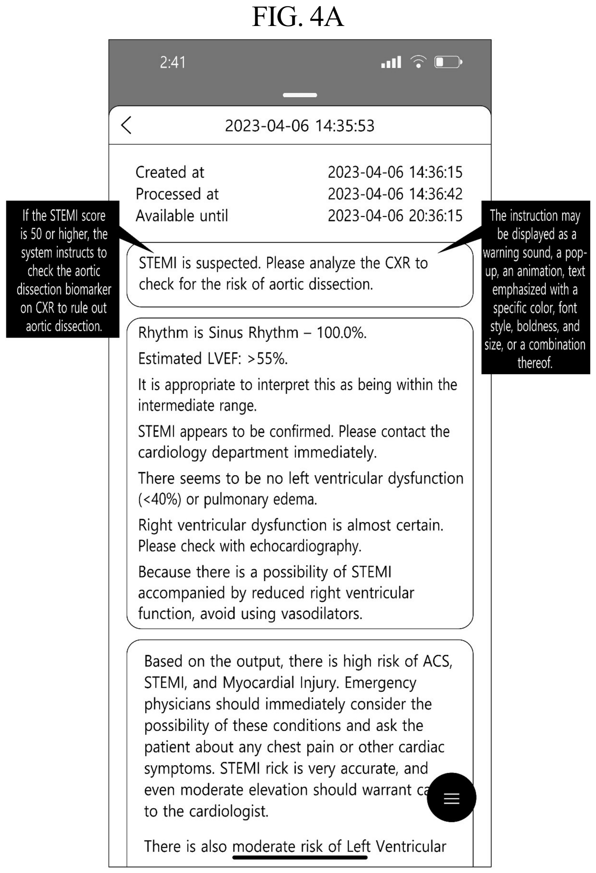

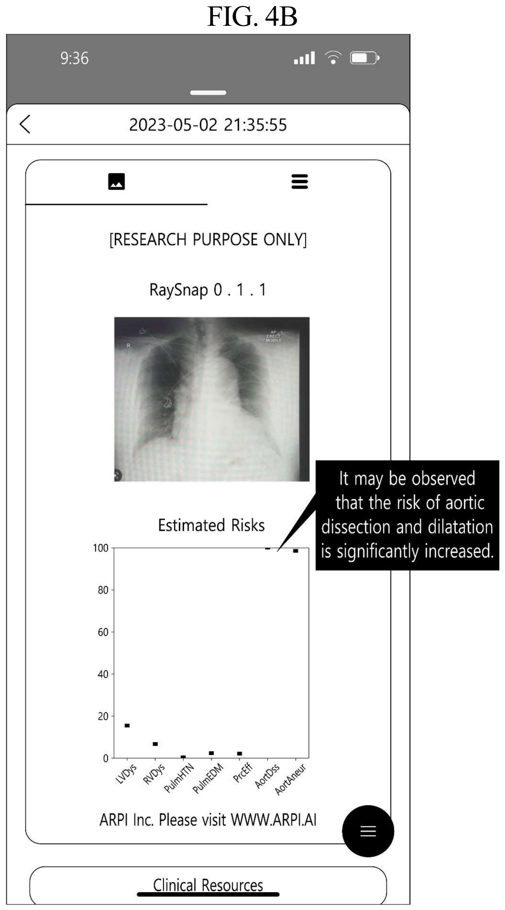

FIGS. 4A and 4B are screens where the apparatus for analyzing test results additionally instructs evaluation of chest radiographic biomarkers after evaluation of ECG biomarkers.

As shown in FIGS. 4A and 4B, the processor of the apparatus for analyzing test results matches a first test result (ECG or chest X-ray) input when providing the analysis with a preset first test biomarker pool, instructs additional verification of a second test (chest X-ray or ECG) biomarker when the matching result corresponds to predefined conditions, combines the first test (ECG or chest X-ray) biomarker and the second test (chest X-ray or ECG) biomarker results, and outputs the combined results.

The predetermined first test or second test biomarker pool may include biomarkers for one or more of left ventricular dysfunction, right ventricular dysfunction, pulmonary hypertension, pulmonary edema, pericardial effusion, aortic dissection, aortic dilatation, (left/right) atrial hypertrophy, (left/right) ventricular hypertrophy, coronary artery calcification/elevated calcium score, coronary artery disease, hypertrophic/dilated/stress cardiomyopathy, and valve stenosis/insufficiency/replacement (AV, MV, TV, PV).

For example, when the ECG biomarker is STEMI/ACS, the corresponding chest X-ray biomarker may be aortic dissection.

When the ECG biomarker is LV dysfunction or pulmonary edema, the corresponding chest X-ray biomarker may be LV dysfunction or pulmonary edema.

When the ECG biomarker is RV dysfunction or pulmonary hypertension, the corresponding chest X-ray biomarker may be RV dysfunction, pulmonary hypertension, or pulmonary embolism.

When the ECG biomarker is a large pericardial effusion, the corresponding chest X-ray biomarker may be a large pericardial effusion.

The operations performed by the apparatus according to embodiments described above may be implemented at least partially as a computer-implementable method or a computer program and may be recorded on a computer-readable recording medium. For example, the operations may be implemented with a program product including a computer-readable medium containing program code, which may be executed by the processor to perform any or all of the described steps, operations, or processes.

The computer may be any device that may be a computing device or may be integrated therewith, such as a desktop computer, a laptop computer, a notebook, a smartphone, or the like. The computer is a device having one or more general-purpose and special-purpose processors, memories, storage spaces, and networking components (either wireless or wired). The computer may execute an operating system, such as, for example, an operating system compatible with Microsoft Windows, Apple OS X or iOS, a Linux distribution, or Google's Android OS.

The computer-readable recording medium includes all types of recording devices in which computer-readable data is stored. Examples of the computer-readable recording medium include ROM, RAM, CD-ROM, magnetic tape, floppy disk, optical data storage devices, and the like. The computer-readable recording medium may also be distributed over networked computer systems so that computer-readable code is stored and executed in a distributed manner. Furthermore, functional programs, codes, and code segments for implementing the present embodiment will be readily understood by those skilled in the art to which the present embodiment belongs.

The present invention described above has been explained with reference to the embodiments shown in the drawings, but these are merely examples, and those skilled in the art will understand that various modifications and variations of the embodiments are possible therefrom. However, such modifications should be considered to be within the technical scope of protection of the present invention. Therefore, the true technical scope of protection of the present invention should be determined by the technical spirit of the appended claims.

### Industrial Applicability

An apparatus and a method for analyzing medical test result images according to an embodiment automatically select and analyze a test area using an artificial neural network and may be utilized in the digital medical industry.

## Claims

1. A method for analyzing medical test result images performed by an apparatus for analyzing medical test result images comprising one or more processors and one or more memories for storing instructions executed by the one or more processors, the method comprising:
selecting, by the processor, a test area to be provided for analysis from medical test result images displayed on a screen using an artificial neural network-based detection model; and
analyzing, by the processor, the selected test area with an artificial neural network-based analysis model to provide results of the analysis,
wherein the medical test result images include one or more of an electrocardiogram (ECG) image and a chest X-ray image.

2. The method of claim 1, wherein the detection model is configured to generate one or more test area candidate boundary boxes and output a tuple including one or more of target test class information and candidate boundary box information.

3. The method of claim 2, wherein the tuple further comprises a confidence score (S), which is a quantitative value indicating whether the class information and the candidate boundary box information correspond to a target class or target boundary box information.

4. The method of claim 3, wherein the screen is obtained by capturing the medical test result images, and the method comprises: calculating a distance (D) between a center of the captured screen and a center of each of the plurality of candidate boundary boxes; and selecting one of the plurality of candidate boundary boxes as a test area of the medical test result images based on the confidence score (S) and the distance (D).

5. The method of claim 4, wherein the method comprises selecting one of the plurality of candidate boundary boxes as a test area of the medical test result images based on a result value (S') of a function [f(S, D)] having the confidence score (S) and the distance (D) as variables, wherein the function [f(S, D)] is selected from one of S, 1/D, and S/D.

6. The method of claim 4, wherein the distance (D) is calculated by one or more of the Euclidean distance, the Manhattan distance, and the Chebyshev distance.

7. The method of claim 2, wherein the class information comprises an index value of a target test, and the candidate boundary box information comprises a numerical value or a combination of numerical values capable of defining a boundary of the box.

8. The method of claim 1, wherein the method comprises changing an area of a plurality of candidate boundary boxes based on drag or resize input by a user.

9. The method of claim 2, wherein the method comprises providing results of the analysis corresponding to a target test using an analysis model assigned to each target test class.

10. The method of claim 1, wherein the method further comprises providing one or more of a test date, a test time, patient identification information, and a patient information memo in addition to the test area.

11. The method of claim 1, wherein the method further comprises, when providing the results of the analysis, providing one or more of a test name, a pending indicator, and a completion notification.

12. The method of claim 1, wherein the method comprises providing a natural language description as results of the analysis, in the providing of the results of the analysis, and generating the natural language description based on biomarker values corresponding to the results of the analysis.

13. The method of claim 12, wherein the natural language description is a rule-based description or a large language model-based description.

14. The method of claim 13, wherein the rule-based description is automatically output when predetermined biomarker values corresponding to results of the analysis are within a set range.

15. The method of claim 13, wherein the large language model-based description is output by creating a prompt based on biomarker values corresponding to the results of the analysis and then inputting the same into the large language model.

16. The method of claim 15, wherein the large language model-based description is generated by generating, according to predefined rules, at least one of the clinical significance of the biomarker and the risk pair derived from measurement values.

17. The method of claim 15, wherein the prompt further includes additional prompts for describing an answer to the associated request.

18. The method of claim 1, wherein the providing of the results of the analysis includes visualizing and providing digital biomarker test results.

19. The method of claim 18, wherein the digital biomarker test results comprise one or more of a name of the digital biomarker, a digital biomarker value, and a distribution of the digital biomarker value.

20. The method of claim 18, wherein the digital biomarker test results comprise one or more of a type of cardiac rhythm and a probability of the type of cardiac rhythm.

21. The method of claim 18, wherein the digital biomarker test results further include a left ventricular ejection fraction.

22. The method of claim 18, wherein the digital biomarker test results further provide ECG thumbnail images, and the method further comprises providing an original ECG image in response to an output request for the thumbnail images.

23. The method of claim 1, wherein the method further comprises requesting consultation between medical professionals regarding medical test results, wherein the requesting of the consultation comprises: providing a buddy list of medical professionals; displaying a consultation availability status based on a stored work schedule of the medical professional's buddy in the list; and in response to a consultation request input, transmitting a consultation request to the requested medical professional's buddy and receiving a response regarding consultation acceptance, wherein the work schedule is electronically updatable, and the consultation availability status is updated when the update of the work schedule is automatically detected by the processor.

24. The method of claim 23, wherein the method further comprises providing an activation indicator indicating the consultation availability status of the medical professional's buddy.

25. The method of claim 23, further comprising providing a user interface for modifying the work schedule.

26. The method of claim 23, wherein the consultation request input includes one or more of patient information and consultation request items.

27. The method of claim 1, wherein the providing of the analysis comprises: matching an input first test result with a preset first test biomarker pool; if the matching result meets predefined conditions, additionally confirming a different second test biomarker; analyzing the first test biomarker and the second test biomarker and combining results; and outputting the combined results.

28. The method of claim 27, wherein the first test is an ECG and the second test is a chest X-ray, and the preset first test or second test biomarker pool includes biomarkers related to one or more of left ventricular dysfunction, right ventricular dysfunction, pulmonary hypertension, pulmonary edema, pericardial effusion, aortic dissection, aortic dilatation, (left/right) atrial hypertrophy, (left/right) ventricular hypertrophy, coronary artery calcification/calcium score elevation, coronary artery disease, hypertrophic/dilated/stress cardiomyopathy, valve stenosis/insufficiency/replacement (AV, MV, TV, PV).

29. The method of claim 28, wherein, when the ECG biomarker is STEMI/ACS, the corresponding chest X-ray biomarker is aortic dissection, when the ECG biomarker is LV dysfunction or pulmonary edema, the corresponding chest X-ray biomarker is LV dysfunction or pulmonary edema, when the ECG biomarker is RV dysfunction or pulmonary hypertension, the corresponding chest X-ray biomarker is RV dysfunction, pulmonary hypertension, or pulmonary embolism, and when the ECG biomarker is a large pericardial effusion, the corresponding chest X-ray biomarker is a large pericardial effusion.

30. An apparatus for analyzing medical test result images, by the method of any one of claims 1 to 29, the apparatus comprising: one or more processors; and one or more memories for storing instructions executed by one or more processors, wherein the one or more processors are configured to provide a first UI screen for acquiring medical test result images and a second UI screen for displaying one or more of a target class and a boundary box for selecting a test area from the acquired medical test result images.
